# FASCICULE DE BREVET EUROPEEN

(11) **EP 0 924 181 B1**
(45) Date de publication et mention de la délivrance du brevet: **03.03.2010**
(21) Numéro de dépôt: 98403066.8
(22) Date de dépôt: 07.12.1998
(51) Int. Cl.: C07C 41/06, B01D 3/38, C07C 7/10

(54) **Procédé d'élimination de composés polaires sur une unité d'éthérification**
Verfahren zum Entfernen von polaren Verbindungen aus einer Etherifizierungseinheit
Process for removing polar compounds from an etherification unit

(30) Priorité: 17.12.1997 FR 9716035
(43) Date de publication de la demande: 23.06.1999
(73) Titulaire: Institut Français du Pétrole, 92852 Rueil-Malmaison Cedex (FR)
(72) Inventeur: Forestière, Alain, 69390 Vernaison (FR); Debuisschert, Quentin, 92500 Rueil-Malmaison (FR); Marache, Pierre, 92500 Rueil-Malmaison (FR); Mikitenko, Paul, 78590 Noisy Le Grand (FR)

(56) Documents cités:
- WO-A-93/16779
- DE-C- 4 330 570
- GB-A- 2 074 035
- US-A- 4 246 417
- US-A- 5 569 790
- US-A- 5 684 212

## Description

La présente invention concerne un procédé d'éthérification d'une charge d'hydrocarbures permettant d'éliminer des impuretés, notamment des traces de composés polaires qui sont contenues dans un fluide, en le mettant en contact avec une phase vapeur générée au moins en partie au cours du procédé ou consécutivement avec une phase vapeur, suivie éventuellement d'une phase solide.

Dans l'expression "composés polaires", on désigne notamment les nitriles, et l'acétone.

De la même façon, le terme "nitriles" regroupe des composés comprenant de l'azote tels que l'acétonitrile, le propionitrile et leurs mélanges éventuels.

L'invention s'intègre bien dans les procédés d'éthérification. En effet, dans de tels procédés il est nécessaire de laver la charge d'hydrocarbures avec de l'eau avant de l'envoyer à la zone de réaction. L'eau de lavage chargée en impuretés peut être purifiée en mettant en oeuvre le procédé selon l'invention.

Dans la suite de la description, le mot "lavage" s'applique aux dispositifs et procédés permettant de laver un second fluide avec un premier fluide, avant d'envoyer le second fluide lavé à un procédé de traitement ultérieur sensible auxdites impuretés, qui peut être par exemple une réaction éthérificative par catalyse acide. Le mot "purification" est employé pour désigner les opérations de traitement du premier fluide qui s'est chargé en impuretés.

Le mot "charge" représente le fluide qui va être soumis à un traitement. Pour le procédé d'éthérification, on utilisera l'expression "charge d'hydrocarbures".

Les procédés d'éthérification sont couramment utilisés pour augmenter la qualité d'indice d'octane des essences. Ces unités traitent typiquement des coupes C₄-C₅ oléfiniques ou des fractions légères d'essence contenant des oléfines tertiaires telles que les isobutènes, les isoamylènes, les isohexènes, les isoheptènes, qui peuvent réagir facilement avec des alcools, par exemple le méthanol, pour donner des éthers méthyliques respectivement éthyliques.

Les procédés d'éthérification peuvent aussi être utilisés intermédiairement pour l'extraction des isooléfines pures à partir de coupes d'hydrocarbures qui les contiennent. Dans ce cas, on produit l'éther par la réaction d'éthérification puis on effectue une décomposition catalysée de l'éther purifié ainsi préalablement isolé. Par exemple, l'isobutylène pur peut être obtenu pour la production de polyisobutylène, du ter-butyl-phénol et ceci par décomposition du méthyl-tertiobutyl-éther (MTBE).

Le procédé de production du MTBE est le processus d'éthérification le plus répandu. La charge hydrocarbonée traitée, une coupe C₄ dans ce cas, contient des oléfines en général et de l'isobutène en particulier. Comme autres procédés d'éthérification qui présentent un intérêt général et qui ont reçu des applications industrielles à grande échelle, on trouve la production de tertioamyl-méthyléther (TAME) par réaction d'isooléfines comprenant 5 atomes de carbone avec du méthanol et la production d'Ethyl-tertiobutyléther (ETBE) obtenu par réaction d'isobutène contenu dans une coupe C₄ avec de l'éthanol.

Toutes ces réactions s'effectuent en général en présence de catalyseurs acides, par exemple et c'est le cas industriel le plus courant, des résines échangeuses d'ions telles que des résines polystyrènes sulfoniques réticulées sous forme acide.

Lorsque les composés polaires, comme les nitriles ou les cétones, sont présents dans la charge d'hydrocarbures de ces procédés d'éthérification, ils réagissent avec le catalyseur, ce qui entraîne une perte des propriétés du catalyseur et sa désactivation.

Afin d'augmenter la durée de vie des catalyseurs, il est connu de l'art antérieur de laver la charge d'hydrocarbures avant son passage sur le catalyseur avec de l'eau déminéralisée en général. Par exemple, avant d'être introduite dans le réacteur où va s'opérer la réaction d'éthérification, une opération classique consiste à faire circuler à contre-courant dans une colonne de lavage, la charge d'hydrocarbures et une eau de lavage dans des conditions opératoires fixées pour que l'eau de lavage se charge en impuretés. A l'issue de cette opération, la charge d'hydrocarbures est épurée en majorité des composés susceptibles d'empoisonner le catalyseur et donc de limiter sa durée de vie. L'eau de lavage chargée en impuretés est envoyée vers un circuit général de traitement des eaux d'une raffinerie comportant une unité d'éthérification.

Cette eau de lavage qui contient les impuretés dont on a débarrassé la charge hydrocarbonée plus des petites quantités d'hydrocarbures, est ensuite traitée par des processus physico-chimiques et/ou bactériologique de façon à la rendre globalement rejetable le cas échéant vers un système d'égout sans que l'on puisse en envisager un quelconque recyclage vers l'unité de lavage située en amont de l'unité d'éthérification dont elle provient. Cette façon d'opérer nécessite l'utilisation d'une quantité importante d'eau de bonne pureté et qui est fournie par une source externe.

Selon le brevet US 5,569,790, la purification de l'eau de lavage de la charge d'hydrocarbures contenant les impuretés est réalisée par une mise en contact liquide-liquide, au moins partiellement avec une partie du raffinat effluent final provenant de l'unité d'éthérification. L'eau purifiée est ensuite recyclée dans la colonne de lavage de la charge. Si un tel procédé offre notamment comme avantage de recycler l'eau, il présente néanmoins l'inconvénient de polluer le raffinat en le chargeant en impuretés ou nitriles. Ceci n'est pas recommandé, le raffinat peut être destiné en aval à d'autres procédés de traitement, tels qu'un procédé d'alkylation. De plus, le degré de purification de l'eau est nécessairement limité, d'une part car le partage des impuretés se fait en faveur de la phase aqueuse, comme le montre le lavage des hydrocarbures, et d'autre part du fait que la quantité relative de raffinat par rapport à l'eau chargée d'impuretés est imposée par le procédé et ne peut être augmentée. Il en résulte que l'eau recyclée est encore impure ce qui diminue l'efficacité du lavage des hydrocarbures. Pour augmenter l'efficacité du lavage, il est alors nécessaire d'injecter un appoint d'eau extérieure dans des quantités relativement importantes. Une telle opération va toutefois à l'encontre du souci d'économiser l'eau, ou de fonctionner en boucle quasi fermée lorsque les sources externes d'eau disponibles sont peu nombreuses. De plus à efficacité de lavage donnée, le débit global de l'eau de lavage vers la colonne de lavage est plus important comparé à celui qui est utilisé dans des procédés fonctionnant à eau perdue (source externe).

L'enseignement technique contenu dans le brevet US 5,684, 212 concerne une amélioration d'un procédé d'éthérification qui comporte une étape où l'eau qui s'est chargée en impuretés en lavant la charge d'hydrocarbures est mise en contact avec un premier flux de vapeur ou un flux de « fuel gas » dans des conditions qui permettent d'éliminer les nitriles de l'eau chargée en impuretés.

Le flux de vapeur ou le « fuel gas » proviennent de sources extérieures au procédé.

Un tel procédé présente notamment l'inconvénient de devoir disposer de sources externes qui ne sont pas toujours disponibles.

L'un des objets de la présente invention est d'utiliser une phase vapeur dans des conditions opératoires données, pour capter les impuretés telles que des composés polaires et plus particulièrement les nitriles (acétonitrile, propionitrile, ...) et l'acétone, qui sont contenus dans une eau de lavage d'une charge d'hydrocarbures destinée par exemple à une réaction d'éthérification.

L'idée consiste notamment à réaliser un procédé en boucle fermée ou quasi-fermée, dans lequel, la phase vapeur utilisée pour l'étape de captation des impuretés est produite au cours du procédé.

La phase vapeur peut être générée par le fluide chargé en impuretés lui-même. Par exemple il est possible d'utiliser la vapeur d'eau générée par le rebouilleur d'une colonne de stripage pour éliminer les impuretés de l'eau de lavage.

Il a aussi été découvert que l'on peut réaliser et/ou parfaire la purification du fluide chargé en impuretés en le faisant passer sur une phase solide choisie pour retenir les impuretés (des composés polaires et éventuellement des hydrocarbures).

La présente invention concerne un procédé d'éthérification d'une charge d'hydrocarbures. Le procédé peut comporter en combinaison au moins les étapes suivantes :
a) on met en contact ladite charge comportant des hydrocarbures (3 à 8 atomes de carbone) et des impuretés telles que des composés polaires, avec une eau de lavage recyclée, pour obtenir une charge d'hydrocarbures appauvrie de la majorité desdites impuretés et une eau de lavage chargée desdites impuretés,
b) on envoie la charge d'hydrocarbures ne comportant pratiquement plus d'impuretés vers une unité d'éthérification,
c) on envoie l'eau de lavage chargée en impuretés vers des étapes de traitement où elle est
   1) purifiée par mise en contact avec une phase vapeur, pour obtenir une phase vapeur chargée en impuretés et une eau de lavage purifiée, ladite phase vapeur étant produite au cours de l'étape c)1),
   2) l'eau de lavage purifiée obtenue à la fin de l'étape 1) est recyclée vers l'étape de lavage de la charge d'hydrocarbures, et
   3) on évacue la phase vapeur chargée en impuretés.

On peut réaliser l'étape a) dans une colonne d'extraction liquide-liquide et/ou l'étape c)1) dans une colonne de distillation.

Les étapes a) et/ou c)1) peuvent être effectuées en faisant circuler les fluides à contre-courant à l'intérieur de la colonne d'extraction-liquide-liquide et de la colonne de purification.

On utilise par exemple au moins une partie de la charge d'hydrocarbures exempte d'impuretés (étape b)) comme agent de refroidissement et/ou on utilise un fluide chaud issu d'une étape du procédé d'éthérification au niveau du rebouilleur de la colonne de purification.

On peut envoyer l'eau chargée en impuretés à une étape de séparation avant l'étape c)1) de mise en contact de manière à éliminer au moins une partie des hydrocarbures les plus légers.

Au cours de l'étape c)1) de mise en contact, on génère par exemple ladite phase vapeur par rebouillage.

On peut introduire l'eau de lavage chargée en impuretés dans une colonne de purification et on peut faire circuler à contre-courant une phase vapeur issue d'un rebouilleur couplé à la colonne de purification.

On envoie au moins ladite phase vapeur chargée en impuretés à une étape de condensation et de séparation de manière à produire au moins une phase essentiellement aqueuse contenant des impuretés et une phase liquide comportant essentiellement des hydrocarbures On produit par exemple une phase gaz contenant des hydrocarbures et une partie des impuretés, et on envoie ladite phase gaz à une étape d'élimination sous une forme gazeuse.

On purge au moins une partie de la phase essentiellement aqueuse. On utilise par exemple au moins une partie de l'eau de lavage purifiée pour chauffer l'eau de lavage chargée en impuretés, avant l'étape de mise en contact.

On peut opérer l'étape c)1) dans un domaine de température compris entre 60 et 210 °C et un domaine de pression variant entre 0,02 MPa et 2 MPa absolu.

L'eau de lavage purifiée est par exemple envoyée à l'issue de l'étape c)1) vers une étape supplémentaire de traitement où elle est mise en contact avec une phase solide capable de retenir les impuretés.

On peut injecter lors de l'étape c)1) une base ou un acide dilués pour maintenir le pH dans un domaine sensiblement neutre.

On envoie par exemple la phase vapeur chargée en impuretés et issue de l'étape de contact c)1) vers une étape de condensation et de séparation à l'issue desquelles, on obtient une phase aqueuse comportant des impuretés, utilisée au moins en partie comme reflux pour l'étape c)1).

On purge par exemple une partie au moins de la phase aqueuse.

Par rapport à l'art antérieur, le procédé selon l'invention présente notamment les avantages suivants:
***** il permet de fonctionner en boucle quasi fermée, entre une colonne de lavage d'une charge hydrocarbure avant une unité de traitement utilisant une eau de lavage, et une colonne de purification de l'eau de lavage chargée en impuretés ; on dispose ainsi d'une grande flexibilité sur le débit d'eau de lavage et on peut donc augmenter le taux d'élimination des impuretés,
* l'appoint et la purge d'eau de lavage sur la boucle fermée sont très faibles, comparés à un procédé de lavage conventionnel à eau perdue de l'art antérieur,
* le degré de purification est élevé, l'eau purifiée pouvant être directement recyclée vers la colonne de lavage; on éliminera au moins 60 % des composés polaires présents dans la charge d'hydrocarbures en fonction du débit d'eau recyclée utilisée et de la nature des composés.

D'autres avantages et caractéristiques selon l'invention seront mieux compris à la lecture de la description d'un exemple d'application du procédé selon l'invention, donné à titre illustratif et nullement limitatif pour la purification d'une eau de lavage d'une charge d'hydrocarbures avant son traitement dans une unité d'éthérification où :
- la figure 1 schématise un circuit de purification de l'eau de lavage de la charge, et
- la figure 2 montre un exemple d'application du circuit de la figure 1 en amont d'une unité d'éthérification.

La charge d'hydrocarbures peut comporter des hydrocarbures de 3 à 8 atomes de carbone, par exemple typiquement des C₄, C₅, mais aussi des C₆, C₇, C₈, et des impuretés telles que des composés polaires précédemment cités (nitriles, acétone).

La charge d'hydrocarbures devant être lavée des impuretés qu'elle contient est envoyée par un conduit 1 en fond d'une colonne de lavage 2. A l'intérieur de cette colonne de lavage 2, elle est mise en contact, par exemple à contre-courant, avec une eau de lavage introduite par exemple en tête de colonne par un conduit 3. Les conditions opératoires de cette opération de lavage, notamment la pression P et la température T, sont choisies de façon à réaliser l'extraction des composés polaires et/ou des composés solubles dans l'eau par l'eau de lavage, et obtenir une charge d'hydrocarbures débarrassée au moins en majorité des impuretés.

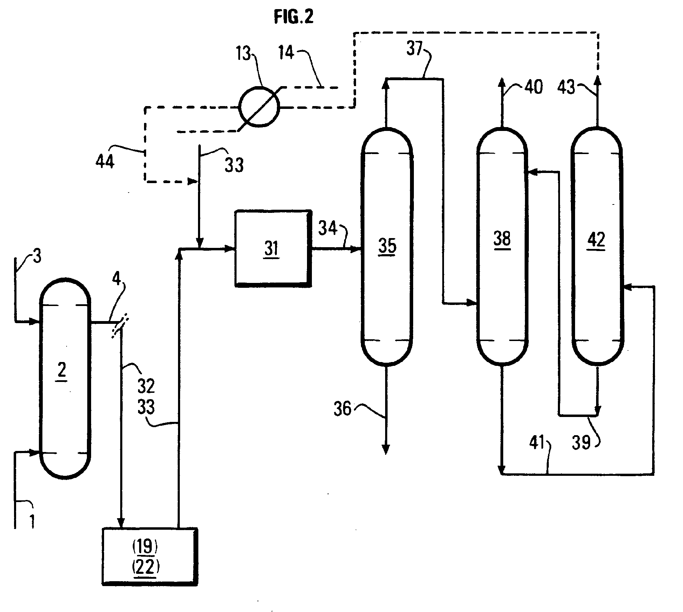

La charge d'hydrocarbures épurée des impuretés est évacuée par un conduit 4 en tête de la colonne de lavage, et envoyée par exemple vers une unité de traitement U₁. Un exemple d'unité de traitement (partie II), par exemple une unité d'éthérification est donné brièvement à la figure 2.

L'eau de lavage qui s'est chargée en impuretés est extraite en fond de colonne de lavage 2 par un conduit 5, et envoyée vers un circuit de traitement ou de purification (partie III de la figure). Au niveau de ce circuit elle est régénérée en mettant en oeuvre les étapes du procédé selon l'invention, avant d'être recyclée vers la colonne de lavage 2.

Une ligne 3b permet l'introduction un appoint d'eau dans la colonne de lavage 2 de la charge d'hydrocarbures.

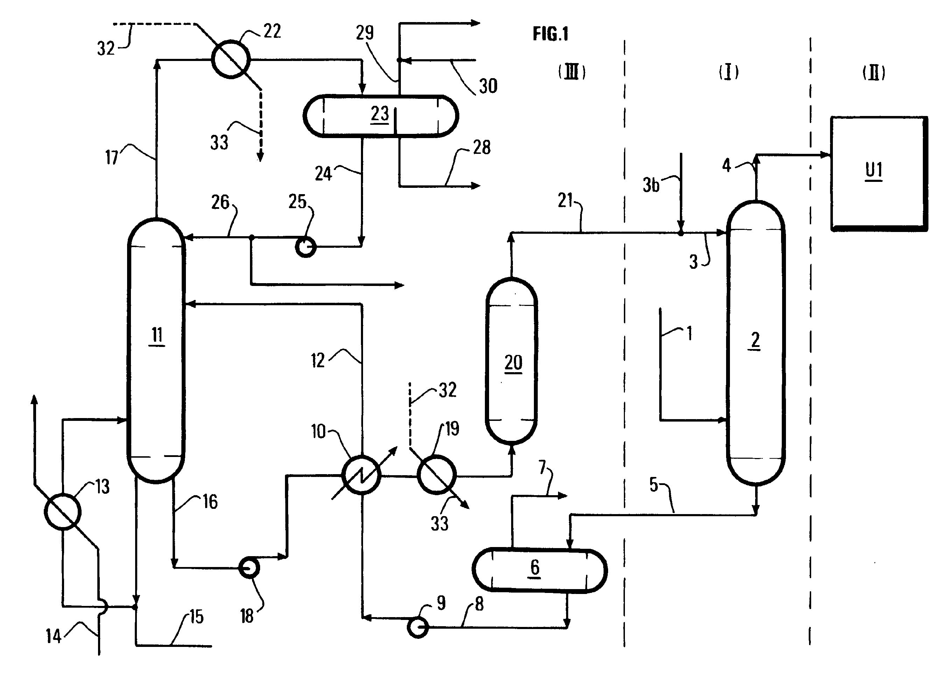

La partie III de la figure 1 schématise un exemple de réalisation pour le circuit de purification de l'eau de lavage chargée en impuretés et issue de la colonne de lavage 2.

L'eau de lavage chargée en composés polaires est envoyée par un conduit 5 de la colonne de lavage 2 vers un ballon tampon 6. Une partie des hydrocarbures les plus légers est évacuée du ballon par un conduit 7, et le reste de l'eau de lavage chargée en composés polaires et appauvrie en hydrocarbures les plus légers est évacuée en fond de ballon tampon 6 par un conduit 8. L'eau chargée en impuretés est envoyée à l'aide d'une pompe 9, et après passage dans un échangeur de chaleur 10, dans une colonne de purification 11. Elle est par exemple introduite en tête de la colonne de purification 11, par un conduit 12.

En fonction des niveaux de pression respectifs des colonnes 2 et 11, il est possible d'envoyer directement l'eau de lavage du fond de colonne 2 vers la colonne de purification sans utiliser de ballon tampon et de pompe.

Cette colonne de purification 11 est pourvue dans sa partie inférieure d'un rebouilleur 13 ayant notamment pour fonction d'initier un flux vapeur, par vaporisation partielle de liquide. Un fluide ayant une température suffisante pour vaporiser partiellement le liquide du fond de colonne alimente le rebouilleur 13 par une ligne 14. La valeur du pH du liquide du fond de colonne peut être contrôlée par un appoint automatique d'acide ou de base dilués, ajoutés par un conduit 15.

L'eau de lavage épurée en majorité des impuretés est extraite en fond de colonne de purification 11 par un conduit 16, et la phase vapeur qui s'est chargée en impuretés (nitriles, acétone et/ou éventuellement d'hydrocarbures) lors de la circulation à contre-courant, est évacuée en tête de colonne par un conduit 17.

L'eau purifiée extraite par le conduit 16 est reprise par une pompe 18, et envoyée vers l'échangeur de chaleur 10 où elle échange des calories pour réchauffer l'eau de lavage chargée en impuretés provenant du ballon 6. L'eau purifiée est ensuite refroidie dans un échangeur 19 avant d'être éventuellement introduite dans un dispositif 20 permettant de parfaire sa purification.

L'eau purifiée issue du dispositif 20 est extraite par un conduit 21, et recyclée vers le conduit 3 d'introduction de l'eau de lavage dans la colonne 2 de lavage de la charge d'hydrocarbures.

Le dispositif 20 peut être formé d'une enceinte comprenant une phase solide, par exemple du charbon actif, une résine ou encore un adsorbant, qui présentent comme particularité de retenir les impuretés résiduelles.

La phase vapeur chargée en impuretés évacuée par le conduit 17, est envoyée, à travers un condenseur 22, vers un ballon 23 dans lequel elle est séparée au moins en trois effluents:
* Une phase essentiellement aqueuse et comportant une partie des impuretés est évacuée par un conduit 24 en fond de ballon 23. Cette phase est reprise par une pompe 25. Une première partie de cette phase aqueuse est envoyée par un conduit 26 pour servir de reflux dans la colonne de purification 11, alors qu'une seconde partie est envoyée vers une ligne de purge 27 de manière à éliminer l'excès éventuel d'eau et une partie des impuretés.
* Une phase composée essentiellement d'hydrocarbures est évacuée du ballon 23 par un conduit 28. Cette phase peut être reprise par une pompe discontinue non représentée sur la figure, et envoyée par exemple pour être utilisée avec la charge d'hydrocarbures avant lavage.
* Une phase gazeuse ou purge gazeuse comportant des hydrocarbures et des impuretés évacuée en tête de ballon par un conduit 29.

Dans le cas où il n'y pas de purge gazeuse, un conduit 30 relié au conduit 29 permet d'introduire une certaine quantité d'azote pour maintenir à l'intérieur du ballon 23 une pression donnée.

Cette manière de procéder offre notamment les avantages suivants :
* la condensation de la vapeur de tête permet de diminuer fortement les pertes d'eau, et donc les appoints d'eau nécessaires,
* de diminuer la perte des hydrocarbures dissous dans l'eau de lavage en les récupérant et les recyclant éventuellement,
* la séparation de la phase gazeuse permet d'éliminer directement les impuretés à la torche, et donc de diminuer le traitement des eaux de rejets.

Sans sortir du cadre de l'invention, afin de réaliser l'étape de purification, il est possible d'utiliser un gaz ou une vapeur différents de la phase vapeur générée par le rebouilleur. On pourra dans le cas où l'on cherche à purifier une eau chargée en impuretés utiliser de la vapeur d'eau introduite en fond de colonne 11. Ce fluide peut alors aussi servir d'appoint dans le circuit de recyclage.

Les conditions de température et de pression qui sont maintenues dans la colonne 11 de purification sont choisies pour permettre l'entraînement au moins de la majorité des impuretés par la vapeur d'eau.

Ainsi, la température à laquelle on opère est comprise entre 60 °C et 210 °C et la pression varie entre 0,02 et 2 MPa absolu.

Selon un mode avantageux de mise en oeuvre du procédé, le pH est contrôlé en fond de colonne de purification 11, par injection d'une base ou d'un acide dilué, afin de le conserver dans un domaine neutre variant par exemple entre 6,5 et 7,5. De cette façon, on limite l'effet de phénomènes indésirables, telle que la corrosion des équipements.

Le taux d'élimination des impuretés de l'eau de lavage en mettant en oeuvre le procédé varie de 85% à 100%.

Le degré de pureté de l'eau purifiée étant élevé, il est possible de la recycler vers l'étape de lavage de la charge d'hydrocarbures sans autre étape de purification importante. Il est aussi possible de parfaire cette étape de purification par passage sur la phase solide du dispositif 20.

En fonctionnant ainsi en boucle quasi-fermée entre la colonne 2 de lavage de la charge et la colonne 11 de purification, on dispose d'une grande flexibilité sur le débit d'eau de lavage. Cette flexibilité permet d'optimiser l'élimination des impuretés de la charge d'hydrocarbures au niveau de la colonne de lavage.

Il est ainsi possible d'éliminer au moins 60% des composés polaires présents dans la charge d'hydrocarbures (second fluide), le taux d'élimination étant fonction, pour un débit donné de cette charge, du débit d'eau recyclée et de la nature des impuretés.

Avantageusement, pour la colonne 11 de purification, le taux de reflux par rapport à la charge se situe entre 2 et 20%.

Le taux de purge par la ligne 29 représente de l'ordre de 0,1 à 5% de l'eau de lavage à traiter, et comprend l'essentiel des impuretés. Le procédé selon l'invention offre comme avantage de rejeter une quantité d'eau chargée en impuretés qui est faible par rapport aux procédés de l'art antérieur.

L'appoint éventuel d'eau sera donc très faible, variable par exemple de 0,1 à 5%.

Afin de limiter les consommations des utilités (condenseur, échangeurs thermiques...), on procédera par exemple à des intégrations thermiques. Par intégration thermique, on entend la possibilité d'utiliser des fluides chaud ou froid provenant d'une unité de traitement à laquelle le procédé selon l'invention est associé, pour servir d'agent de chauffe au niveau du rebouilleur ou d'agent de refroidissement pour le condenseur 22 et/ou l'échangeur refroidisseur 19.

Un exemple du procédé selon l'invention disposé en amont d'une unité d'éthérification en tenant compte de la possibilité de limiter les consommations est donné ci-après.

### Exemple utilisant la possibilité de diminuer la consommation des utilités d'une unité d'éthérification

La figure 2 schématise un exemple d'unité d'éthérification disposée après un circuit de purification tel que décrit à la figure 1. Pour des raisons de clarté et de simplification, seuls les flux matières ont été schématisés, les vannes, échangeurs, ligne de purge, pompes,..... n'ont pas été représentés.

### Exemple de purification de l'eau de lavage de la charge d'hydrocarbures selon le procédé de purification

### Données spécifiques à l'unité d'éthérification

La charge d'hydrocarbures comportant initialement les impuretés est par exemple composée de monooléfines, et de paraffines, contenant chacune de 3 à 8 atomes de carbone, typiquement des C₄, C₅, mais aussi des C₆, C₇, C₈, dans la majorité des cas en quantité supérieure à celle des mono oléfines. Les paraffines peuvent inclure du butane, du propane, de l'isobutane, de l'isopentane ou du n-pentane, des hexanes (iso ou n-).

Les mono oléfines peuvent comporter du propylène, du butène-1, du butène-2, de l'isobutène, du 2-méthyl-2-butène, du 2-métyl-1-butène, du 3-méthyl-1-butène, du 1-pentène, du 2-pentène, du cyclopentène, du cyclohexène. Cette charge peut aussi contenir en faible quantité des dioléfines telles que du 1,3 butadiène et du 1,3 pentadiène, de l'isopropène, du cyclo pentadiène.

Les impuretés ou composés polaires en particulier des nitriles (définis au début de la description), sont présents dans la charge sous la forme de traces dans des proportions variant entre 10 et 500 ppm/poids et plus particulièrement dans l'intervalle 15 à 80 ppm-poids. La charge peut aussi contenir de l'eau dans une quantité variable entre 5 et 500 ppm-poids mesuré en H₂O à saturation. Les impuretés peuvent aussi comprendre des composés oxygénés tels que des alcools, des éthers, des aldéhydes, des cétones et des acides. Comme exemples plus spécifiques pour ces composés, on peut mentionner l'éthanol, le méthanol, l'acétone....

L'acétone peut être présent sous forme de traces variant dans la gamme de 1 à 500 ppm-poids. La charge peut ou non avoir été soumis à un procédé d'hydrogénation sélective pour éliminer les dioléfines avant ou après traitement. Typiquement, la charge peut contenir entre 50 ppm-poids et environ 4 % poids de dioléfines.

### Conditions opératoires du procédé d'éthérification

Le domaine de température pour réaliser l'opération d'éthérification se situe par exemple de 20 à 400 °C, et la gamme de pression entre la pression atmosphérique et 1MPa. Les rapports molaires stoechiométriques de l'alcool aux isooléfines réactives varient, par exemple, dans une gamme allant de 0,2 :1 à environ 10:1 et de préférence entre 0,95 et 1,15. Ces conditions seront choisies en fonction du catalyseur de la zone de réaction.

### Schéma de l'unité d'éthérification

L'unité d'éthérification U₁ comporte une zone de réaction 31 pourvue d'un conduit 33 d'introduction de l'alcool d'appoint tel que du méthanol nécessaire à l'éthérification, un conduit 32 d'arrivée de la charge d'hydrocarbures lavée issue de la colonne de lavage 2 et éventuellement un conduit 44 de recyclage de l'alcool.

Au préalable, la charge d'hydrocarbures lavée est réchauffée en passant à travers le condenseur 22 ou l'échangeur 19 (figure 1), pour lesquels elle joue le rôle d'agent de refroidissement. La zone de réaction comporte un catalyseur choisi pour réaliser la réaction d'éthérification et fonctionne dans des conditions telles que l'on obtient en sortie un mélange formé par les C₄ non convertis et le méthanol, et l'éther produit au cours de la réaction est envoyé par un conduit 34 vers une colonne de distillation 35.

On évacue en fond de colonne de distillation 35 un flux contenant l'éther produit par un conduit 36.

En tête de colonne, on extrait par un conduit 37 les hydrocarbures et le méthanol qui n'ont pas réagi, et on les envoie à une colonne 38 de lavage du raffinat. La colonne de lavage 38 de raffinat est équipée d'un conduit 39 permettant d'introduire l'eau de lavage du raffinat. On évacue par un conduit 40 en tête de colonne 38 un raffinat débarrassé du méthanol et en fond par un conduit 41 une solution formée par l'eau et le méthanol.

Cette solution est envoyée vers une colonne de distillation 42. En fond de la colonne de distillation 42, on évacue l'eau par le conduit 39 avant de la recycler vers la colonne 38 de lavage du raffinat. En tête de colonne de distillation 42, on extrait par un conduit 43 le méthanol sous forme vapeur. Ce dernier peut être envoyé pour servir de moyens de chauffage, avant d'être recyclé vers la zone de réaction 31. Pour cela le méthanol extrait par le conduit 43 passe dans le conduit 14 du rebouilleur 13 (figure 1). Après avoir cédé une partie de sa chaleur pour initier le flux vapeur nécessaire au fonctionnement de la colonne de purification 11 (figure 1), le méthanol refroidi est recyclé par le conduit 44 vers la zone de réaction 31. On peut ajuster la pression au niveau de la colonne de distillation 42 pour obtenir le méthanol à une température suffisante lui permettant de jouer le rôle d'agent de chauffe.

L'exemple numérique donné ci-après permet d'illustrer les avantages offerts par le procédé selon l'invention pour purifier l'eau de lavage de charge.

L'eau de lavage chargée en impuretés est extraite de la colonne de lavage de la charge d'hydrocarbures avec un débit qui peut représenter de l'ordre de 5 à 150 % de la charge d'hydrocarbures, suivant la nature des impuretés de cette charge et leur teneur.

L'eau introduite dans le ballon 6 comporte comme impuretés principales :
⇒ des Hydrocarbures dissous : 200 à 500 ppm poids,
⇒ des nitriles 10 à 300 ppm poids et acétone 10 à 300 ppm poids.

En tête de ballon 6, le débit de gaz composé des hydrocarbures les plus légers varie de 0 à 0,005 % du débit d'eau de lavage à purifier.

En fond de la colonne de purification 11, l'eau extraite purifiée possède des impuretés dont les teneurs sont données ci-après :
⇒ des hydrocarbures dissous < à 10 ppm,
des nitriles < à 3 ppm et acétone < à 3 ppm.

En tête de la colonne de purification, la phase vapeur chargée en impuretés est évacuée avec un débit pouvant aller jusqu'à 0,05 % poids du débit d'eau de lavage à purifier entrant dans la colonne de purification.

Le débit du distillat liquide évacué par le conduit 28 est compris entre 0 et 0,05 % poids du débit d'eau de lavage entrant dans la colonne de purification.

Par la ligne de purge 27, on évacue une quantité d'eau avec un débit variant de 0 à 5 % du débit d'eau de lavage à purifier, et ayant une teneur en impuretés se répartissant comme suit :
⇒ hydrocarbures dissous 200 à 500 ppm poids,
⇒ nitriles variant entre 0,1 % et 10 % poids et acétone compris entre 0,1 et 6 % poids.

## Revendications

1. Procédé d'éthérification d'une charge d'hydrocarbures, **caractérisé en ce qu'**il comporte en combinaison au moins les étapes suivantes :
a) on met en contact une charge comportant des hydrocarbures de 3 à 8 atomes de carbone et au moins une des impuretés du groupe incluant les nitriles et l'acétone, avec une eau de lavage recyclée, pour obtenir une charge d'hydrocarbures appauvrie en majorité desdites impuretés et une eau de lavage chargée en impuretés,
b) on envoie ladite charge d'hydrocarbures ne comportant pratiquement plus d'impuretés vers une unité d'éthérification,
c) on envoie l'eau de lavage chargée en impuretés vers des étapes de traitement où elle est
1) purifiée par mise en contact avec une phase vapeur, pour obtenir une phase vapeur chargée en impuretés et une eau de lavage purifiée, ladite phase vapeur étant produite au cours de l'étape c)1),
2) l'eau de lavage purifiée obtenue à la fin de l'étape c)1) est recyclée vers l'étape de lavage de la charge d'hydrocarbures, et
3) on évacue la phase vapeur chargée en impuretés.

2. Procédé d'éthérification selon la revendication 1 **caractérisé en ce que** l'on réalise l'étape a) dans une colonne d'extraction liquide-liquide et/ou l'étape c)1) dans une colonne de distillation.

3. Procédé d'éthérification selon la revendication 1 **caractérisé en ce que** l'on réalise les étapes a) et/ou c)1) par une circulation à contre-courant.

4. Procédé d'éthérification selon la revendication 1 **caractérisé en ce que** on envoie l'eau chargée en impuretés à une étape de séparation avant l'étape c)1) de mise en contact de manière à éliminer au moins une partie des hydrocarbures les plus légers.

5. Procédé d'éthérification selon l'une des revendications 1 à 4, **caractérisé en ce que** l'on utilise au moins une partie de la charge d'hydrocarbures exempte d'impuretés (étape b)) comme agent de refroidissement.

6. Procédé selon la revendication 1 **caractérisé en ce que** lors de l'étape c)1) de mise en contact, on génère ladite phase vapeur par rebouillage.

7. Procédé selon la revendication 1 **caractérisé en ce que** l'on introduit l'eau de lavage chargée en impuretés dans une colonne de purification et on fait circuler à contre-courant la phase vapeur.

8. Procédé selon l'une des revendications 1 à 7 **caractérisé en ce que** l'on envoie au moins ladite phase vapeur chargée en impuretés à une étape de condensation et de séparation de manière à produire au moins une phase essentiellement aqueuse contenant des impuretés et une phase liquide comportant essentiellement des hydrocarbures.

9. Procédé selon la revendication 8 **caractérisé en ce que** l'on produit au moins une phase gaz contenant des hydrocarbures et une partie des impuretés, et on envoie ladite phase gaz à une étape d'élimination.

10. Procédé selon la revendication 8 **caractérisé en ce que** l'on recycle au moins une fraction de la phase essentiellement aqueuse pour l'utiliser comme reflux lors de l'étape de mise en contact.

11. Procédé selon la revendication 8 **caractérisé en ce que** l'on purge au moins une partie de la phase essentiellement aqueuse.

12. Procédé selon la revendication 1 **caractérisé en ce que** l'on utilise au moins une partie de l'eau de lavage purifiée pour chauffer l'eau de lavage chargée en impuretés.

13. Procédé selon l'une des revendications 1 à 12 **caractérisé en ce que** l'on opère l'étape c)1) dans un domaine de température compris entre 60 et 210 °C et un domaine de pression variant entre 0,02 MPa et 2 MPa absolu.

14. Procédé selon l'une des revendications 1 à 13 **caractérisé en ce que** l'on injecte lors de l'étape c)1) une base ou un acide dilués pour maintenir le pH dans un domaine sensiblement neutre.

15. Procédé selon les revendications 1 à 14 **caractérisé en ce que** l'eau de lavage purifiée est envoyée à l'issue de l'étape c)1) vers une étape supplémentaire de traitement où elle est mise en contact avec une phase solide capable de retenir les impuretés.

## Claims

1. An etherification process for a hydrocarbon load, **characterised in that** it comprises, in combination, at least the following steps:
a) bringing a load comprising hydrocarbons with 3 to 8 carbon atoms and at least one of the impurities of the group including nitriles and acetone into contact with recycled washing water to obtain a hydrocarbon load that is mostly depleted of said impurities and a washing water that is loaded with impurities,
b) sending said hydrocarbon load that practically no longer contains impurities to an etherification unit,
c) sending the washing water that is loaded with impurities to treatment steps where it is
1) purified by being brought into contact with a vapour phase, to obtain a vapour phase that is loaded with impurities and a purified washing water, said vapour phase being produced during step c)1),
2) the purified washing water that is obtained at the end of step c)1) is recycled to the hydrocarbon load washing step, and
3) the vapour phase that is loaded with impurities is evacuated.

2. The etherification process according to Claim 1 **characterised in that** step a) is carried out in a liquid-liquid extraction column and/or step c)1) is carried out in a distillation column.

3. The etherification process according to Claim 1 **characterised in that** steps a) and/or c)1) are carried out by countercurrent circulation.

4. The etherification process according to Claim 1 **characterised in that** the water that is loaded with impurities is sent to a separation step before the contacting step c)1) so as to eliminate at least a portion of the lightest hydrocarbons.

5. The etherification process according to one of claims 1 to 4, **characterised in that** at least one portion of the hydrocarbon load that is free of impurities (step b)) is used as a cooling agent.

6. The process according to Claim 1 **characterised in that**, during the contacting step c)1), said first vapour phase is generated by boiling.

7. The process according to Claim 1 **characterised in that** the washing water loaded with impurities is fed into a purification column and the vapour phase is made to circulate in a countercurrent manner.

8. The process according to one of claims 1 to 7 **characterised in that** at least said vapour phase that is loaded with impurities is sent to a condensation and separation step in order to produce at least one essentially aqueous phase containing the impurities and one liquid phase essentially comprising hydrocarbons.

9. The process according to Claim 8 **characterised in that** at least one gas phase containing hydrocarbons and a portion of the impurities is produced, and said gas phase is sent to an elimination step.

10. The process according to Claim 8 **characterised in that** at least one fraction of the essentially aqueous phase is recycled in order to be used as a reflux during the contacting step.

11. The process according to Claim 8 **characterised in that** at least one portion of the essentially aqueous phase is drawn off.

12. The process according to Claim 1 **characterised in that** at least one portion of the purified washing water is used to heat the washing water that is loaded with impurities.

13. The process according to one of claims 1 to 12 **characterised in that** step c)1) is operated in a temperature range between 60 and 210°C and a pressure range varying between 0.02 MPa and 2 MPa absolute.

14. The process according to one of claims 1 to 13 **characterised in that**, during-step c)1), a diluted base or acid is injected to keep the pH within a substantially neutral range.

15. The process according to claims 1 to 14 **characterised in that** the purified washing water is sent, at the end of step c)1), to an additional treatment step where it is brought into contact with a solid phase that is capable of retaining the impurities.

## Patentansprüche

1. Verfahren zur Veretherung einer Kohlenwasserstoffbeschickung, **dadurch gekennzeichnet, dass** es in Kombination mindestens die folgenden Schritte umfasst:
a) Inkontaktbringen einer Beschickung, die Kohlenwasserstoffe mit 3 bis 8 Kohlenstoffatomen und mindestens eine der Verunreinigungen der Gruppe, die Nitrile und Aceton beinhaltet, umfasst, mit einem recycelten Waschwasser, um eine Kohlenwasserstoffbeschickung, die überwiegend verarmt an Verunreinigungen ist, und ein Waschwasser, das mit den Verunreinigungen beladen ist, zu erhalten,
b) Schicken der Kohlenwasserstoffbeschickung, die praktisch keine Verunreinigungen mehr enthält, in eine Veretherungseinheit,
c) Schicken des Waschwassers, das mit Verunreinigungen beladen ist, zu Behandlungsschritten, in denen
1) es durch Inkontaktbringen mit einer Dampfphase gereinigt wird, um eine mit Verunreinigungen beladene Dampfphase und ein gereinigtes Waschwasser zu erhalten, wobei die Dampfphase im Verlauf von Schritt c)1) produziert wird,
2) das gereinigte Waschwasser, das am Ende von Schritt c)1) erhalten wird, zum Schritt des Waschens der Kohlenwasserstoffbeschickung recycelt wird, und
3) die Dampfphase, die mit Verunreinigungen beladen ist, abgeleitet wird.

2. Verfahren zur Veretherung nach Anspruch 1, **dadurch gekennzeichnet, dass** der Schritt a) in einer Flüssig-flüssig-Extraktionssäule und/oder der Schritt c)1) in einer Destillationssäule ausgeführt werden.

3. Verfahren zur Veretherung nach Anspruch 1, **dadurch gekennzeichnet, dass** die Schritte a) und/oder c)1) mittels Gegenstromzirkulation ausgeführt werden.

4. Verfahren zur Veretherung nach Anspruch 1, **dadurch gekennzeichnet, dass** das mit Verunreinigungen beladene Wasser zu einem Trennungsschritt vor dem Schritt c)1) des Inkontaktbringens geschickt wird, um mindestens einen Teil der leichtesten Kohlenwasserstoffe zu entfernen.

5. Verfahren zur Veretherung nach einem der Ansprüche 1 bis 4, **dadurch gekennzeichnet, dass** mindestens ein Teil der Kohlenwasserstoffbeschickung, die frei von Verunreinigungen ist (Schritt b)), als Kühlmittel verwendet wird.

6. Verfahren nach Anspruch 1, **dadurch gekennzeichnet, dass** während des Schritts c)1) des Inkontaktbringens, die Dampfphase durch Wiedererhitzen erzeugt wird.

7. Verfahren nach Anspruch 1, **dadurch gekennzeichnet, dass** das mit Verunreinigungen beladene Waschwasser in eine Reinigungssäule eingeführt wird und die Dampfphase im Gegenstrom zirkuliert wird.

8. Verfahren nach einem der Ansprüche 1 bis 7, **dadurch gekennzeichnet, dass** mindestens die mit Verunreinigungen beladene Dampfphase zu einem Kondensations- und Trennungsschritt geschickt wird, um mindestens eine im Wesentlichen wässrige Phase zu produzieren, die Verunreinigungen enthält, und eine liquide Phase, die im Wesentlichen Kohlenwasserstoffe umfasst.

9. Verfahren nach Anspruch 8, **dadurch gekennzeichnet, dass** mindestens eine Gasphase produziert wird, die Kohlenwasserstoffe und einen Teil der Verunreinigungen enthält, und die Gasphase zu einem Entfernungsschritt geschickt wird.

10. Verfahren nach Anspruch 8, **dadurch gekennzeichnet, dass** mindestens eine Fraktion der im Wesentlichen wässrigen Phase recycelt wird, um sie als Rückfluss während des Schritts des Inkontaktbringens zu verwenden.

11. Verfahren nach Anspruch 8, **dadurch gekennzeichnet, dass** mindestens ein Teil der im Wesentlichen wässrigen Phase geklärt wird

12. Verfahren nach Anspruch 1, **dadurch gekennzeichnet, dass** mindestens ein Teil des gereinigten Waschwassers verwendet wird, um das erste mit Verunreinigungen beladene Waschwasser zu erwärmen.

13. Verfahren nach einem der Ansprüche 1 bis 12, **dadurch gekennzeichnet, dass** der Schritt c)1) in einem Temperaturbereich zwischen 60 und 210 °C und einem Druckbereich, der zwischen 0,02 MPa und 2 MPa absolut variiert, durchgeführt wird.

14. Verfahren nach einem der Ansprüche 1 bis 13, **dadurch gekennzeichnet, dass** während des Schritts c)1) eine verdünnte Base oder eine verdünnte Säure injiziert wird, um den pH-Wert in einem im Wesentlichen neutralen Bereich zu halten.

15. Verfahren nach den Ansprüchen 1 bis 14, **dadurch gekennzeichnet, dass** das gereinigte Waschwasser am Schluss des Schritts c)1) zu einem zusätzlichen Behandlungsschritt geschickt wird, bei dem es mit einer festen Phase in Kontakt gebracht wird, die in der Lage ist, die Verunreinigungen zurückzuhalten.
